# EUROPEAN PATENT APPLICATION

(11) **EP 4 756 034 A1**
(43) Date of publication of application: **10.06.2026**
(21) Application number: 24848648.2
(22) Date of filing: 23.05.2024
(51) Int. Cl.: C12Q 1/68, C12M 1/00, C12M 1/34, G01N 33/48, G01N 33/53

(54) **DETECTION METHOD, PRESENTATION DEVICE, DETECTION DEVICE, DETECTION SYSTEM, DETECTION PROGRAM, AND RECORDING MEDIUM**

(30) Priority: 31.07.2023 JP 2023125051
(71) Applicant: ARKRAY, Inc., Kyoto-shi, Kyoto 601-8045 (JP)
(72) Inventor: UCHIYAMA, Makoto, Kyoto-shi, Kyoto 602-0008 (JP); HARAKO, Sora, Kyoto-shi, Kyoto 602-0008 (JP)
(74) Representative: MFG Patentanwälte Meyer-Wildhagen Meggle-Freund Gerhard PartG mbB
(86) International application number: PCT/JP2024/019099
(87) International publication number: WO 2025/027987

(57) **Abstract**

A detection method has: a measuring step that measures expression levels of plural nucleic acid molecules contained in each of plural specimens; an evaluating step that, by using an evaluation algorithm that evaluates properties of the specimens based on expression levels of nucleic acid molecules, evaluates properties of each of the plural specimens based on the expression levels of the nucleic acid molecules that were measured in the measuring step, and determines evaluation values; and a detecting step that assigns ranks to the evaluation values of the plural specimens with respect to high/low levels, and, based on correlation between the ranks and the evaluation values of the plural specimens, detects an abnormality that has arisen in processes up to determination of the evaluation values.

## Description

### Technical Field

The present disclosure relates to a detection method, a presentation device, a detection device, a detection system, a detection program and a recording medium.

### Background Art

Japanese Patent No. 7021097 discloses a disease affection judging device having a sample data acquiring unit and an affection judging unit. The sample data acquiring unit acquires sample data including the expression levels of plural types of miRNA in a sample originating from a living body. By using a trained model, the affection judging unit outputs results of judging affection of plural diseases at plural regions of the body from the acquired sample data. The trained model is a trained model that has been obtained in advance by machine learning using training data that includes plural sample data having items for identifying the absence/presence of affection of plural diseases in plural regions of the body, and that can judge affection of the plural diseases that include plural malignant diseases or plural benign diseases, including cases of being affected by plural diseases.

International Publication No. 2021/132547 discloses a testing method that carries out testing of a disease by using a disease marker, and includes a specimen data acquiring step and a determining step. The specimen data acquiring step acquires marker data expressing results of measuring a disease marker within a bodily liquid specimen taken from a subject, and preparation data expressing the conditions under which the bodily liquid specimen was prepared. The determining step determines the absence/presence of affection by a disease at the subject by inputting the marker data and the preparation data acquired in the specimen data acquiring step to a trained model that has been trained by machine learning the correlation between, on the one hand, a set of the marker data expressing the results of measurement of the disease marker in the bodily liquid specimen and the preparation data expressing the preparation conditions of the bodily liquid specimen, and on the other hand, the absence/presence of affection of the disease in the subject from which the bodily liquid specimen was obtained.

### SUMMARY OF INVENTION

### Technical Problem

Here, in a case of using an evaluation algorithm that evaluates properties of each of plural specimens based on the expression levels of plural nucleic acid molecules, abnormalities may arise in the evaluation algorithm itself or in the processes leading up to usage of the evaluation algorithm. Accordingly, it is desirable to be able to easily detect unacceptable abnormalities that arise in the processes up until the completion of evaluation of properties of each of plural specimens. Examples of these abnormalities are abnormalities that arise in following cases (1) through (3) for example.
(1) A case in which the evaluation algorithm that is the standard for evaluation deteriorates for some reason.
(2) A case in which the plural specimens that are the objects of evaluation are different than objects of evaluation that were assumed for the evaluation by the evaluation algorithm that is the standard for evaluation.
(3) A case in which, due to improper treatment in a pretreatment before measurement or the like, an abnormality arises in the results of measurement of the expression levels of the nucleic acid molecules.

A topic of the present disclosure is to provide a detection method, a detection device, a detection system, a detection program and a recording medium that, in a case in which, by using an evaluation algorithm that evaluates properties of a specimen based on expression levels of nucleic acid molecules, properties of each of plural specimens are evaluated based on expression levels of nucleic acid molecules that have been measured in advance and evaluation values are determined, can easily detect unacceptable abnormalities that arise in processes up until the determining of the evaluation values.

### Solution to Problem

A detection method relating to an aspect of the present disclosure has: a measuring step that measures expression levels of plural nucleic acid molecules contained in each of plural specimens; an evaluating step that, by using an evaluation algorithm that evaluates properties of the specimens based on expression levels of nucleic acid molecules, evaluates properties of each of the plural specimens based on the expression levels of the nucleic acid molecules that were measured in the measuring step, and determines evaluation values; and a detecting step that assigns ranks to the evaluation values of the plural specimens with respect to high/low levels, and, based on correlation between the ranks and the evaluation values of the plural specimens, detects an abnormality that has arisen in processes up to determination of the evaluation values.

### Advantageous Effects of Invention

In accordance with the present disclosure, unacceptable abnormalities that arise in processes up to the determination of evaluation values can be easily detected in a case in which, by using an evaluation algorithm that evaluates properties of a specimen based on expression levels of nucleic acid molecules, properties of each of plural specimens are evaluated based on expression levels of nucleic acid molecules that have been measured in advance, and evaluation values are determined.

### BRIEF DESCRIPTION OF DRAWINGS

Fig. 1 is a flowchart illustrating an example of respective steps of a detection method relating to a present embodiment.
Fig. 2 is a graph illustrating an example of correlation between ranks and evaluation values of plural specimens.
Fig. 3 is a graph illustrating, in addition to an example of correlation between ranks and evaluation values of plural specimens, an example of judgment results and accompanying information that accompanies the judgment results.
Fig. 4 is a block drawing illustrating an example of a computer that functions as a detection device of the present embodiment.
Fig. 5 is a block drawing illustrating an example of functional structures of the detection device of the present embodiment.

### DESCRIPTION OF EMBODIMENTS

Examples of embodiments relating to the technique of the present disclosure are described hereinafter. Note that structural elements and processings whose operations, effects and functions have the same workings are denoted by the same reference numerals throughout all of the drawings, and redundant description may be omitted as appropriate. The respective drawings merely illustrate the technique of the present disclosure schematically to the extent that it can be sufficiently understood. Accordingly, the technique of the present disclosure is not limited to only the illustrated examples. Further, in the present embodiment, there are cases in which description of structures that are not directly related to the present disclosure and well-known structures is omitted.

### <Detection Method 10>

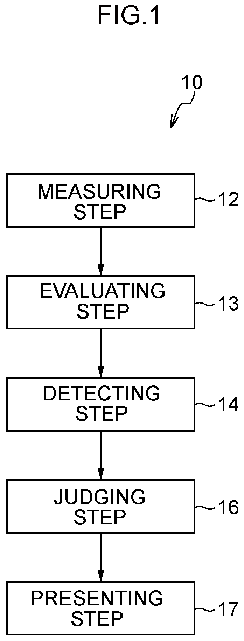

A detection method 10 relating to the present embodiment is described first. Fig. 1 is a schematic drawing illustrating an example of the respective steps of the detection method 10 relating to the present embodiment.

The detection method 10 is a method of measuring expression levels of plural nucleic acid molecules contained in each of plural specimens, evaluating properties of each of the plural specimens based on the expression levels and determining evaluation values, and detecting abnormalities that have arisen in processes up until the evaluation values are determined. Examples of specimens that are objects of measurement for which the expression levels of plural nucleic acid molecules are measured are bodily fluids (e.g., blood, blood serum, urine, tears, saliva, sweat, semen, lymphatic fluid, tissue fluid, body cavity effusion (e.g., pleural effusion, ascitic fluid), cerebral fluid, amniotic fluid, vaginal secretions, nasal mucus), tissues, and cells. The subject from which the specimen is taken may be a human or may be a non-human animal. Examples of non-human animals are non-human mammals (monkeys, dogs, cats, mice, rats, rabbits, cows, horses, pigs, sheep), birds (chickens, quail), and the like.

Micro RNA is an example of the nucleic acid molecules. Note that the nucleic acid molecules may be small RNA other than micro RNA, or may be another type of RNA or DNA. Moreover, the nucleic acid molecules do not have to be nucleic acid molecules structured from only ATGCU bases. Specific examples are nucleic acids that have been modified such as by DNA/RNA methylation or that have been edited such as by A-to-I RNA editing. In this way, various nucleic acid molecules can be subjects to which the present detection method is applied.

As illustrated in Fig. 1, the detection method 10 specifically has a measuring step 12, an evaluating step 13, a detecting step 14, a judging step 16 and a presenting step 17. In the present embodiment, as an example, the measuring step 12, the evaluating step 13, the detecting step 14, the judging step 16 and the presenting step 17 are executed in that order.

Note that, because the detection method 10 is a method having the measuring step 12, the evaluating step 13, the judging step 16 and the presenting step 17, the detection method 10 can also be called a measurement method, an evaluation method, a judgment method or a presentation method. Further, in a case in which inspection or analysis or the like is carried out by the judgment in the judging step 16, the detection method 10 can also be called an inspection method or an analysis method. The respective steps of the detection method 10 are described hereinafter.

### <Measuring Step 12>

The measuring step 12 is a step of measuring the expression levels of plural nucleic acid molecules that are contained in each of plural specimens. Specifically, in the measuring step 12, by using a next generation sequencer (hereinafter called NGS upon occasion) that is a measurement device, plural nucleic acid molecules that are contained in each of plural specimens are measured, and the base sequence of each of the nucleic acid molecules is specified. Next, by counting, in each base sequence, the number of each specified nucleic acid molecule, the number of reads of the nucleic acid molecule at the NGS is determined. This number of reads of a nucleic acid molecule is the number of the nucleic acid molecules contained in the specimen, and corresponds to the expression level (specifically, the absolute expression level) of the nucleic acid molecule. Note that the number of reads of a nucleic acid molecule that is outputted by an NGS may be normalized, and the expression level may be measured as an expression level that is relative (i.e., the relative expression level). Means such as RPM (Read Per Million) normalization, or normalization using internal standard small RNA can be used as the normalizing means. In this way, the expression level of a nucleic acid molecule may be the relative expression level thereof.

Note that, other than a next generation sequencer, a quantitative PCR or a flow cytometer or the like can be used as the measurement device, and it suffices to be able to measure the expression levels of plural nucleic acid molecules.

In an NGS, it is possible to measure the expression levels of plural nucleic acid molecules of plural specimens all at once. Namely, in the measuring step 12, the expression levels of plural nucleic acid molecules of plural specimens are measured in a same process.

Note that there are cases in which, before the measuring step 12 is executed, a pretreatment (e.g., centrifugal separation) is carried out on the specimen. In the present embodiment, if a pretreatment is carried out, the same treatment is carried out on the plural specimens. Namely, in the present embodiment, a pretreatment is carried out on the plural specimens in a same process.

### <Evaluating Step 13>

The evaluating step 13 is a step that, by using an algorithm that evaluates properties of a specimen based on expression levels of nucleic acid molecules (hereinafter called evaluation algorithm), evaluates properties of each of plural specimens based on the expression levels of the nucleic acid molecules that were measured in the measuring step 12, and determines evaluation values.

In the evaluating step 13, first, the expression levels of the nucleic acid molecules measured in the measuring step 12 are analyzed. Specifically, the number of molecules per type of nucleic acid molecule is totaled up. Note that, in a case of using blood (specifically, blood serum) of a human as a specimen, there are, for example, 500 types of micro RNA that are the nucleic acid molecules that are measured.

In the evaluating step 13, next, properties of the specimens are evaluated based on the totaled numbers of molecules (specifically, for example, the distribution of the numbers of molecules that were totaled up for each type of nucleic acid molecule). For example, disease prediction of the donor who provided the specimen is an example of the evaluation of properties of a specimen. Disease prediction can be carried out by using a disease predicting algorithm as the evaluation algorithm.

Specifically, machine learning is carried out in advance by using, as training data, the numbers of molecules of the respective micro RNA that are the subjects, and information regarding the absence/presence of a disease, and a learning model that serves as a disease predicting algorithm is constructed. Then, by using the totaled-up numbers of molecules as the input factors, evaluation values (evaluation scores) of disease prediction can be determined as the output factors by using the learning model.

Note that the evaluation of properties of a specimen is not limited to disease prediction, and may be basic biological analysis, regressive prediction, detection of various types of abnormalities, or the like.

### <Detecting Step 14>

In the detecting step 14, the evaluation values of the plural specimens (hereinafter called specimen group upon occasion) are ranked in accordance with their high/low levels, and, based on the correlation between the ranks and the evaluation values of the plural specimens, abnormalities that have arisen in the processes up to the determining of the evaluation values are detected. Hereinafter, abnormalities that are objects of detection in the detecting step 14 are called "targeted abnormalities" upon occasion. Note that the specimen group is not limited to plural specimens on which measurement of the expression levels of plural nucleic acid molecules has been carried out all at once at a measurement device such as an NGS. The specimen group may be, for example, plural specimens in which such measurement has been carried out continuously by a same process and by a same measurement device. Accordingly, specimen group also includes plural specimens on which such measurement has been carried out over plural times.

An example of ranking the plural specimens in order from the highest evaluation value is described hereinafter, but the plural specimens may be ranked in order from the lowest evaluation value. Namely, the ranking may be ranking in order from the highest evaluation value, or may be ranking in order from the lowest evaluation value.

Further, the ranking may be, for example, the application of absolute numerical values when ordering the plural specimens. If ranking is carried out in this way, for example, when 50 specimens are ranked in order from the highest evaluation value, the rank of the evaluation value that is 20th from highest is the rank "20". Or, for example, the ranking may be the application of ratios that are relative numerical values expressing the relative positions of the plural specimens. If ranking is carried out in this way, for example, when 50 specimens are ranked in order from the highest evaluation value, the rank of the evaluation value that is 20th from highest is the rank "40"%.

The targeted abnormalities that are detected in the detecting step 14 are abnormalities that arise in processes up to the determining of the evaluation values. Accordingly, targeted abnormalities include abnormalities originating in the specimen that is the object of evaluation, and abnormalities arising in a pretreatment before execution of the measuring step 12, or in the measuring step 12 or the evaluating step 13. For example, abnormalities that arise in following cases (1) through (3) are examples of targeted abnormalities.
(1) A case in which the evaluation algorithm that is the standard for evaluation deteriorates for some reason.
(2) A case in which the specimen group that is the object of evaluation is different than objects of evaluation that were assumed for the evaluation by the evaluation algorithm that is the standard for evaluation.
(3) A case in which, due to there having been an improper treatment in a pretreatment executed before the measuring step 12, or in the measuring step 12 or the like, an abnormality arises in the results of measurement of the expression levels of the nucleic acid molecules.

An example of above (1) is data drift (also called feature drift or covariate drift) that is due to the statistical distribution of the input data that is the training data at the time of creating the learning model and the statistical distribution of the input data at the time of evaluation becoming offset due to some change. An example of above (2) is a case in which, in evaluation in accordance with a disease predicting algorithm that assumes that the frequency of diseased patients is low, a specimen group that is diseased patients for the most part is used as the object of evaluation. An example of above (3) is a case in which there was an improper treatment in the treating of the specimens, which was needed for the measuring step 12, by a worker, or there was an abnormality with the reagent used in treating the specimens.

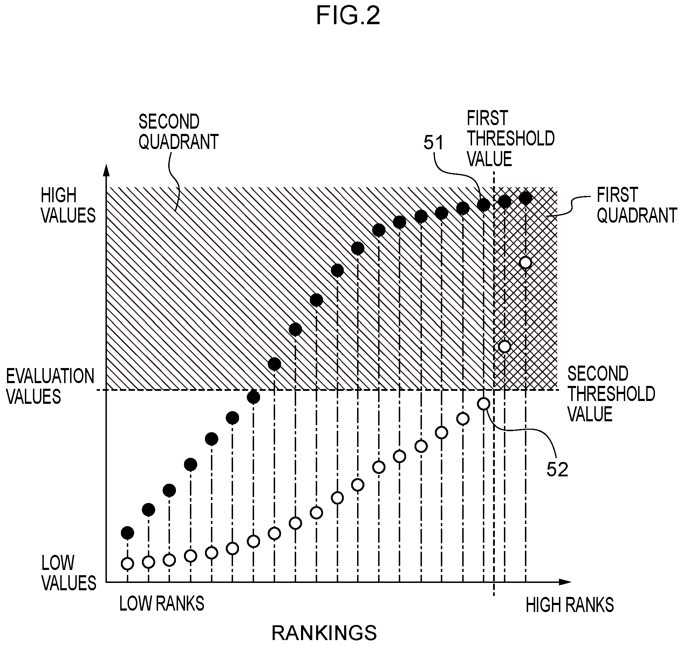

In the detecting step 14, for example, a case in which the evaluation value of any of the specimens, whose ranks are lower than a first threshold value, is a value that is greater than or equal to a second threshold value is detected as a targeted abnormality. Here, in the graph shown in Fig. 2, evaluation values are on the vertical axis, and the high/low ranks of the evaluation values are on the horizontal axis. Further, in the example illustrated in Fig. 2, the first threshold value is a value of the rank of 10% for example.

Here, for example, a threshold value that is a judgment index in the judging step 16 is used as the second threshold value. Namely, as will be described later, in a case in which, in the judging step 16 that carries out prediction of the absence/presence of a disease for example, it is judged that there is a disease if the evaluation value is greater than or equal to a predetermined threshold value, that threshold value is set as the second threshold value.

On the other hand, in the judging step 16 that carries out prediction of the absence/presence of a disease for example, a rank that is less than the lowest rank at which a judgment that there is a disease is expected is used as the first threshold value. For example, in a case in which a judgment that there is a disease in the judging step 16 is assumed for ranks above 10%, the value of 10% is set as the first threshold value for example.

As described above, because the first threshold value and the second threshold value are set, if the evaluation value of any specimen whose rank is lower than the first threshold value is a value that is greater than or equal to the second threshold value, a specimen that is judged to have a disease in the judging step 16 (i.e., a specimen that is greater than or equal to the second threshold value) exists at a rank at which a judgment in the judging step 16 that there is a disease was not expected (i.e., a rank that is lower than the first threshold value). Therefore, in the detecting step 14, it can be detected that an abnormality has arisen in processes up to the determining of the evaluation value.

As illustrated in Fig. 2, in the specimen group shown by the black dots, the evaluation value (refer to reference numeral 51) of a specimen whose rank is lower than the first threshold value is a value that is greater than or equal to the second threshold value. In contrast, in the specimen group shown by the white dots, the evaluation value (refer to reference numeral 52) of a specimen whose rank is lower than the first threshold value is a value that is less than the second threshold value. Accordingly, in the example illustrated in Fig. 2, the evaluation group shown by the black dots corresponds to a case in which the evaluation value of any of the specimens whose rank is lower than the first threshold value is a value that is greater than or equal to the second threshold value. Namely, this corresponds to a case in which a specimen that is judged to have a disease in the judging step 16 (i.e., a specimen greater than or equal to the second threshold value) exists at a rank at which a judgment that there is a disease in the judging step 16 was not supposed (i.e., a rank lower than the first threshold value). Therefore, in the detecting step 14, the specimen group shown by the black dots is detected as a targeted abnormality.

On the other hand, in the specimen group shown by the white dots, the evaluation values of all of the specimens whose rank is lower than the first threshold value are values that are less than the second threshold value. Namely, a specimen that is judged to not have a disease in the judging step 16 (i.e., a specimen less than the second threshold value) exists at a rank at which a judgment that there is a disease in the judging step 16 was not supposed (i.e., a rank lower than the first threshold value). Therefore, in the detecting step 14, the specimen group shown by the white dots is detected as having normal evaluation values. Namely, in the detecting step 14, a targeted abnormality is not detected with respect to the specimen group shown by the white dots.

Note that, in the case of the graph of Fig. 2, it can also be said that a targeted abnormality is detected in a case in which any of the dots is included in the region marked by the diagonal lines in Fig. 2 (hereinafter called the second quadrant upon occasion).

Note that the detection method described by using Fig. 2 is effective for specimen groups in which there are hardly any specimens whose evaluation values are high values.

Further, in the above description, it is detected that there is an targeted abnormality in a case in which any of the dots is included in the second quadrant. However, it may be detected that there is an targeted abnormality in a case in which plural dots are included in the second quadrant.

Moreover, it may be detected that there is an targeted abnormality in a case in which the dot (evaluation value) whose rank is nth or is n% is a value that is greater than or equal to the second threshold value.

It is desirable that the second threshold value that is used in the detecting step 14 be determined in accordance with, for example, the contents of the pretreatment caried out before execution of the measuring step 12, the measurement device used in the measuring step 12, the contents of judging in the judging step 16, or the contents of evaluating in the evaluating step 13.

On the other hand, it is desirable that the first threshold value that is used in the detecting step 14 be a fixed value, because it is a rank and it is a relative index that does not depend on the contents executed in the respective steps. Note that, when supposing a case in which the specimen group that is the object of evaluation is different than the object of evaluation that was expected for the evaluation by the evaluation algorithm that is the standard for evaluation, it is desirable that the first threshold value change in accordance with the attributes of the specimen group that is the object of evaluation.

Further, plural patterns of the combination of the first threshold value and the second threshold value used in the detecting step 14 may be readied in advance. In this case, in the detecting step 14, the user selects one pattern from the plural patterns, and the targeted abnormality is detected by using this pattern. Moreover, in the detecting step 14, it is possible to detect a targeted abnormality by deciding upon at least the first threshold value based on information relating to the origin source from which the specimen group was taken. Note that, because at least the first threshold value is based on information relating to the origin source from which the specimen group was taken, the user can set the first threshold value to an arbitrary value.

For example, in a case in which the specimens are blood (specifically, blood serum) of humans, information relating to the origin source from which the specimen group was taken is information expressing what the origin source is (e.g., medical checkups, a cancer center).

If the origin source from which the specimen group was taken is an origin source at which there is the tendency for the evaluation values to be high values, a combination in which the first threshold value is set to be relatively low is selected.

### <Judging Step 16>

The judging step 16 is a step of carrying out, on the specimen group, a judgment relating to the specimens based on the evaluation values determined by the evaluating step 13. This judging step 16 is executed in a case in which the detecting step 14 has detected no targeted abnormalities. Accordingly, the judging step 16 is not executed if a targeted abnormality is detected in the detecting step 14. Note that, in this case, the user (i.e., the person executing the detection method) may be presented with information expressing that a targeted abnormality has been detected for example.

An example of a judgment relating to a specimen is the prediction of the absence/presence of a disease in the donor who provided the specimen. For example, if the evaluation value determined in the evaluating step 13 is greater than or equal to the second threshold value (see Fig. 2), it can be judged in the judging step 16 that there is a disease. In the case of the graph illustrated in Fig. 2, it is judged that there is a disease for a donor who provided a specimen corresponding to a dot existing in the region adjacent to the second quadrant at the right side thereof (the first quadrant in Fig. 2).

As described above, in a case in which a targeted abnormality is detected in the detecting step 14, the judging step 16 is not executed. However, the present disclosure is not limited to this. For example, even in a case in which a targeted abnormality is detected in the detecting step 14, the judging step 16 may be executed, for example, in a case of obtaining results of judgment as reference data. In this case, for example, upon detection of a targeted abnormality, the user may be presented with information expressing that judgment was carried out.

### <Presenting Step 17>

The presenting step 17 is a step of presenting the correlation between the ranks and the evaluation values of the plural specimens. In the presenting step 17, for example, the correlation of the ranks, which were given to the respective specimens of the specimen group in the detecting step 14, and the evaluation values is presented.

Specifically, the distribution (e.g., the graph illustrated in Fig. 2) of the ranks and the evaluation values of the plural specimens, with respect to the first threshold value that is set for the ranks and the second threshold value that is set for the evaluation values, is presented in the presenting step 17.

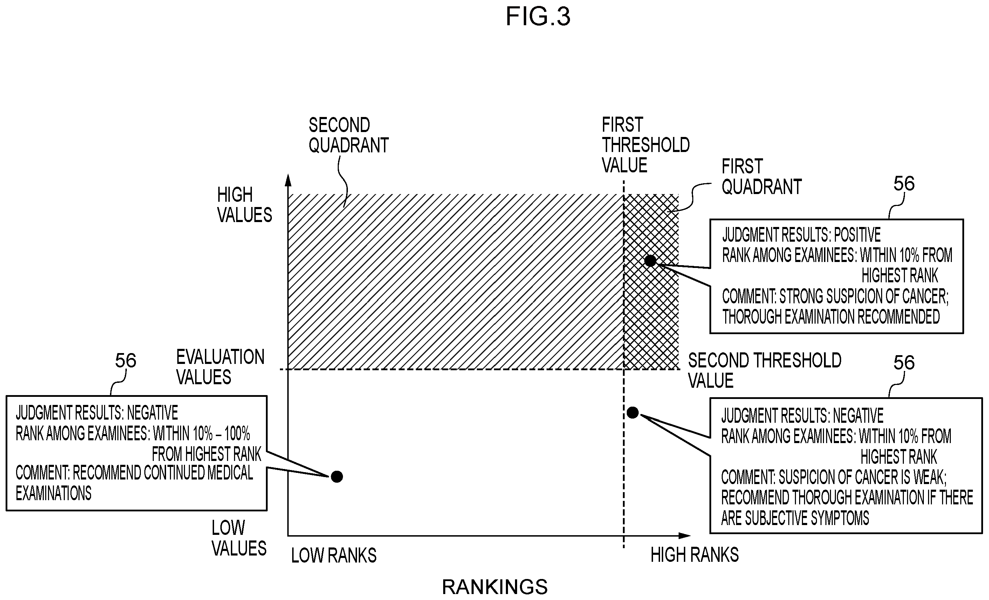

Further, in addition to the correlation of the ranks, which were given to the respective specimens of the specimen group in the detecting step 14, and the evaluation values, judgment results 56 (e.g., the graph illustrated in Fig. 3) of the judging step 16 may be presented in the presenting step 17. In the example illustrated in Fig. 3, prediction of the absence/presence of cancer (specifically, prediction of whether positive or negative) and accompanying information that accompanies the results of judgment (e.g., comments on the rank, within the specimen group, of the medical examinee (corresponding to the donor of the specimen), and on the results of judgment) are presented as the results of judgment that predict the absence/presence of a disease.

Examples of the presentation method are a method of presenting by displaying on a display portion, a method of printing onto a recording medium such as paper, and the like. Examples of the display portion are a liquid crystal display and an organic EL (Electro Luminescence) display.

Examples of the presentees to which the correlation is presented are the donors of the specimens, and, if it is predicted that there is a disease, the doctor or the like who will treat the disease.

### <Detection System 20>

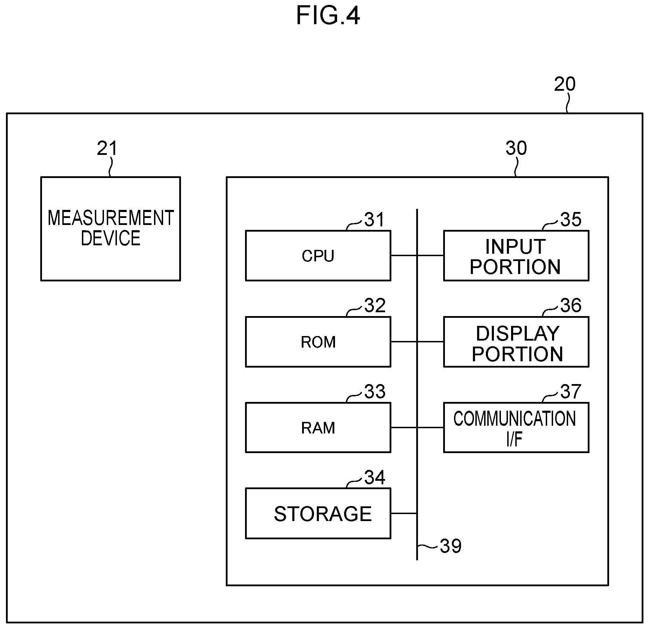

A detection system 20 serving as a system that executes the above-described detection method 10 is described next. As illustrated in Fig. 4, the detection system 20 has a measurement device 21 and a detection device 30.

### <Measurement Device 21>

The measurement device 21 is an example of the measurement unit, and is a device that executes the above-described measuring step 12. Namely, the measurement device 21 measures the expression levels of plural nucleic acid molecules contained in each of plural specimens. An NGS for example is used as the measurement device 21.

### <Detection Device 30>

The detection device 30 is an example of the evaluation unit, and is an example of the detector. The detection device 30 is a device that executes the above-described evaluating step 13 and the above-described detecting step 14. Namely, by using an evaluation algorithm that evaluates properties of a specimen based on expression levels of nucleic acid molecules, the detection device 30 evaluates properties of each of plural specimens based on expression levels of nucleic acid molecules that have been measured in advance, and determines evaluation values. Moreover, the detection device 30 assigns ranks to the evaluation values of the plural specimens with respect to their high/low levels, and, based on the correlation between the ranks and the evaluation values of the plural specimens, detects an abnormality that has arisen in the processes up until the determination of the evaluation values.

Further, the detection device 30 executes the above-described judging step 16 and presenting step 17. Namely, based on the evaluation values that the detection device 30 itself determined, the detection device 30 carries out a judgment relating to the specimens of the specimen group. Moreover, the detection device 30 carries out evaluation based on the expression levels of the nucleic acid molecules that were measured in advance, and determines evaluation values, and assigns ranks to the evaluation values of the plural specimens with respect to their high/low levels, and functions as a presentation device that presents the correlation between the ranks and the evaluation values of the plural specimens. Specifically, the detection device 30 presents a distribution (e.g., the graph illustrated in Fig. 2) of the ranks and the evaluation values of the plural specimens, with respect to the first threshold value that is set for the ranks and the second threshold value that is set for the evaluation values.

The detection device 30 has the functions of a computer, and, as illustrated in Fig. 4, has a CPU (Central Processing Unit) 31, a ROM (Read Only Memory) 32, a RAM (Random Access Memory) 33, a storage 34, an input portion 35, a display portion 36 and a communication interface (I/F) 37. These respective structural sections are connected so as to be able to communicate with one another via bus 39.

The CPU 31 (an example of the processor) is a central computing processing unit, and executes various programs and controls respective sections. Namely, the CPU 31 reads-out a program from the ROM 32 or the storage 34, and executes the program by using the RAM 33 as a workspace. The CPU 31 carries out control of the above-described respective structures, and various types of computing processings, in accordance with programs stored in the ROM 32 or the storage 34. Note that the CPU 31 is an example of the processor.

The ROM 32 stores various programs and various data. The RAM 33 temporarily stores programs and data as a workspace. The storage 34 is structured by an HDD (Hard Disk Drive) or an SSD (Solid State Drive), and various programs, including the operating system, and various data are recorded therein.

In the present embodiment, for example, a detection program for causing execution of the detection processing that carries out the above-described detection method 10 is recorded in the storage 34. The detection program may be one program, or may be a program group structured by plural programs or modules. Note that the detection program may be recorded in the ROM 32. The ROM 32 or the storage 34 functions as an example of a non-transitory recording medium.

Examples of the processor are not limited to the above-described CPU that is a generic processor, and, for example, may be a dedicated processor structured by circuits designed exclusively for executing specific processings for example. Further, examples of the processor are not limited to cases of being structured by one processor, and may be formed by plural processors that are provided at positions physically remote from one another cooperating with one another.

The input portion 35 includes a pointing device such as a mouse, and a keyboard, and is used in order to carry out various types of input. Further, the input portion 35 receives, as input, information of the expression levels of the plural nucleic acid molecules that were measured by the measurement device 21. Moreover, the input portion 35 receives, as input, the above-described information relating to the origin source, and information that are used in the evaluating step 13 and the detecting step 14 such as the contents of evaluation and the subjects of evaluation.

The display portion 36 is a liquid crystal display for example, and displays various information. At the detection device 30, information expressing that a targeted abnormality has been detected can be presented to the user through the display portion 36.

At the detection device 30, the correlation between the ranks and the evaluation values of the plural specimens can be presented to the user through the display portion 36. Accordingly, in the present embodiment, the display portion 36 can be understood as being an example of the presentation device. Note that a touch panel type display may be employed as the display portion 36, and the display portion 36 may be made to function as the input portion 35.

The communication interface 37 is an interface for communicating with other equipment, and standards such as, for example, Ethernet^{™}, FDDI (Fiber Distributed Data Interface), or Wi-Fi^{™} are used thereat.

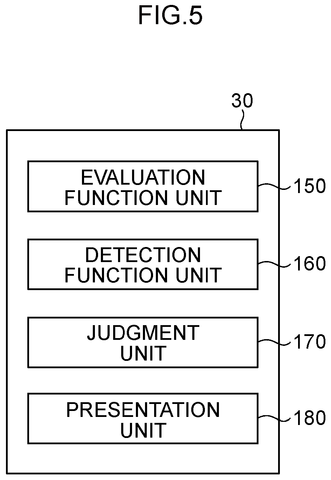

As illustrated in Fig. 5, at the detection device 30, by executing the detection program, the CPU 31 functions as an evaluation function unit 150, a detection function unit 160, a judgment unit 170, and a presentation unit 180.

The evaluation function unit 150 executes the above-described evaluating step 13. Namely, by using an evaluation algorithm that evaluates properties of a specimen based on expression levels (expression levels) of nucleic acid molecules, the evaluation function unit 150 evaluates properties of each of plural specimens based on the expression levels of the nucleic acid molecules that were measured at the measurement device 21, and determines evaluation values (refer to the above-described evaluating step 13).

The detection function unit 160 executes the above-described detecting step 14. Namely, the detection function unit 160 assigns ranks to the evaluation values of the respective specimens in the specimen group in accordance with their high/low levels, and detects targeted abnormalities based on the correlation between the ranks and the evaluation values of the plural specimens (refer to the above-described detecting step 14).

The judgment unit 170 executes the judging step 16. Namely, based on the evaluation values determined by the evaluation function unit 150, the judgment unit 170 carries out, on the specimen group, a judgment relating to the specimens (refer to the above-described judging step 16).

The presentation unit 180 executes the above-described presenting step 17. Namely, the presentation unit 180 presents the correlation between the ranks and the evaluation values of the plural specimens (refer to the above-described presenting step 17).

Note that, in the present embodiment, the detection system 20 has the measurement device 21 and the detection device 30, but the detection system 20 may be structured by a single device. In this case, the single device functions as an example of the measurement unit, the evaluation unit and the detector.

Further, the detection device 30 may be structured by plural devices. For example, the detection device 30 may be structured by plural (e.g., four) devices that separately execute the above-described evaluating step 13, detecting step 14, judging step 16 and presenting step 17.

### <Operation and Effects of Present Embodiment>

In the present embodiment, as described above, in the detecting step 14, the evaluation values of plural specimens are ranked based on their high/low levels, and targeted abnormalities (i.e., abnormalities that have arisen in the processes up to determining the evaluation values) are detected based on the correlation (see Fig. 2) between the ranks and the evaluation values of the plural specimens.

Due thereto, the distribution of the evaluation values of the plural specimens (i.e., the specimen group) is clear, and targeted abnormalities that are unacceptable can be detected easily.

Moreover, in the present embodiment, in the detecting step 14, a case in which the evaluation value of any specimen, whose rank is lower than the first threshold value, is a value greater than or equal to the second threshold value for example, is detected as a targeted abnormality.

Due thereto, for example, in a case in which any dot is included in the second quadrant of the graph illustrated in Fig. 2, such a case can be detected as a targeted abnormality, and targeted abnormalities that are unacceptable can be detected easily.

In the measuring step 12, the expression levels of plural nucleic acid molecules are measured for plural specimens in a same process.

Because evaluation of the properties of the specimens is executed based on the expression levels of plural nucleic acid molecules that have been measured under the same measuring conditions, the reliability of the evaluation values increases, and targeted abnormalities can be detected highly accurately in the detecting step 14.

Further, in the present embodiment, if a targeted abnormality is not detected in the detecting step 14, in the judging step 16, a judgment relating to the specimens is carried out on the specimen group based on the evaluation values that were determined by the evaluating step 13.

In the judging step 16, execution of a judgment whose results have low reliability can be suppressed as compared with a case of always carrying out, on a specimen group, a judgment relating to the specimens regardless of the results of detection of the detecting step 14.

Further, in the present embodiment, in the detecting step 14, targeted abnormalities are detected by using one pattern from a combination of plural patterns of the first threshold value and the second threshold value.

Therefore, the pattern that is used in detecting targeted abnormalities can be selected in accordance with the specimens that are the objects of detection, and targeted abnormalities can be detected highly accurately in the detecting step 14.

Moreover, in the present embodiment, in the detecting step 14, the first threshold value is decided upon based on information relating to the origin source from which the specimen group was taken, and targeted abnormalities are detected.

Because the first threshold value that is used in detecting targeted abnormalities is decided upon in accordance with the information relating to the origin source, targeted abnormalities can be detected highly accurately in the detecting step 14.

Further, in the present embodiment, the detection device 30 carries out evaluation based on expression amounts of nucleic acid molecules that were measured in advance, and determines evaluation values, and assigns ranks to the evaluation values of plural specimens based on their high/low levels, and presents the correlation between the ranks and the evaluation values of the plural specimens. Due thereto, the presentee to which the correlation is presented can detect targeted abnormalities based on the correlation.

Specifically, the detection device 30 presents the distribution (e.g., the graph illustrated in Fig. 2) of the ranks and the evaluation values of the plural specimens, with respect to the first threshold value that is set for the ranks and the second threshold value that is set for the evaluation values. Due thereto, the presentee can easily detect targeted abnormalities that are unacceptable.

The present disclosure is not limited to the above-described embodiments, and various modifications, changes and refinements can be made thereto within a scope that does not depart from the gist of the present disclosure. The present disclosure may be structured by appropriately combining a plurality of the above-described modified examples.

### <Notes>

### (Aspect 1)

A detection method comprising:
a measuring step that measures expression levels of a plurality of nucleic acid molecules contained in each of a plurality of specimens;
an evaluating step that, by using an evaluation algorithm that evaluates properties of the specimens based on expression levels of nucleic acid molecules, evaluates properties of each of the plurality of specimens based on the expression levels of the nucleic acid molecules that were measured in the measuring step, and determines evaluation values; and
a detecting step that assigns ranks to the evaluation values of the plurality of specimens with respect to high/low levels, and, based on correlation between the ranks and the evaluation values of the plurality of specimens, detects an abnormality that has arisen in processes up to determination of the evaluation values.

### (Aspect 2)

The detection method of Aspect 1, wherein, in the detecting step:
the evaluation values of the plurality of specimens are ranked in order from highest, and
a case in which an evaluation value of any specimen, whose rank is lower than a first threshold value, is a value that is greater than or equal to a second threshold value is detected as the abnormality.

### (Aspect 3)

The detection method of Aspect 1 or Aspect 2, wherein, in the measuring step:
the expression levels are measured for the plurality of specimens in a same process.

### (Aspect 4)

The detection method of any one of Aspects 1 through 3, further comprising a judging step that, in a case in which the abnormality is not detected in the detecting step, carries out, on the plurality of specimens, a judgment relating to the specimens based on the evaluation values determined by the evaluating step.

### (Aspect 5)

The detection method of any one of Aspects 2 through 4, wherein:
there are a plurality of patterns of combinations of the first threshold value and the second threshold value, and
in the detecting step, the abnormality is detected by using one pattern of the plurality of patterns.

### (Aspect 6)

The detection method of Aspect 5, wherein, in the detecting step, the first threshold value is decided upon based on information relating to an origin source from which the plurality of specimens were taken, and the abnormality is detected.

### (Aspect 7)

A presentation device that, based on expression levels of nucleic acid molecules contained in each of a plurality of specimens, assigns ranks to evaluation values, by which properties of the specimens are evaluated, with respect to high/low levels, and presents a correlation between the ranks and the evaluation values of the plurality of specimens.

### (Aspect 8)

The presentation device of Aspect 7, wherein the presentation device presents a distribution of the ranks and the evaluation values of the plurality of specimens, with respect to a first threshold value that is set for the ranks and a second threshold value that is set for the evaluation values.

### (Aspect 9)

The presentation device of Aspect 7 or 8, wherein the presentation device presents accompanying information that accompanies judgment results of judgments relating to the specimens that were carried out based on the evaluation values.

### (Aspect 10)

A detection device comprising a processor, wherein the processor:
by using an evaluation algorithm that evaluates properties of specimens based on expression levels of nucleic acid molecules, evaluates properties of each of a plurality of specimens based on expression levels of the nucleic acid molecules that have been measured in advance, and determines evaluation values; and
assigns ranks to the evaluation values of the plurality of specimens with respect to high/low levels, and, based on correlation between the ranks and the evaluation values of the plurality of specimens, detects an abnormality that has arisen in processes up to determination of the evaluation values.

### (Aspect 11)

A detection system comprising:
a measurement unit that measures expression levels of a plurality of nucleic acid molecules contained in each of a plurality of specimens;
an evaluation unit that, by using an evaluation algorithm that evaluates properties of the specimens based on expression levels of nucleic acid molecules, evaluates properties of each of the plurality of specimens based on the expression levels of the nucleic acid molecules that were measured at the measurement unit, and determines evaluation values; and
a detector that assigns ranks to the evaluation values of the plurality of specimens with respect to high/low levels, and, based on correlation between the ranks and the evaluation values of the plurality of specimens, detects an abnormality that has arisen in processes up to determination of the evaluation values.

### (Aspect 12)

A detection program for causing a computer to execute detecting processing that:
by using an evaluation algorithm that evaluates properties of specimens based on expression levels of nucleic acid molecules, evaluates properties of each of a plurality of specimens based on expression levels of the nucleic acid molecules that have been measured in advance, and determines evaluation values; and
assigns ranks to the evaluation values of the plurality of specimens with respect to high/low levels, and, based on correlation between the ranks and the evaluation values of the plurality of specimens, detects an abnormality that has arisen in processes up to determination of the evaluation values.

### (Aspect 13)

A non-transitory recording medium on which is recorded a detection program for causing a computer to execute detecting processing that:
by using an evaluation algorithm that evaluates properties of specimens based on expression levels of nucleic acid molecules, evaluates properties of each of a plurality of specimens based on expression levels of the nucleic acid molecules that have been measured in advance, and determines evaluation values; and
assigns ranks to the evaluation values of the plurality of specimens with respect to high/low levels, and, based on correlation between the ranks and the evaluation values of the plurality of specimens, detects an abnormality that has arisen in processes up to determination of the evaluation values.

The disclosure of Japanese Patent Application No. 2023-125051 filed on July 31, 2023 is, in its entirety, incorporated by reference into the present specification. All publications, patent applications and technical standards mentioned in the present specification are incorporated by reference into the present specification to the same extent as if each publication, patent application or technical standard were specifically and individually indicated to be incorporated by reference.

## Claims

1. A detection method comprising:
a measuring step that measures expression levels of a plurality of nucleic acid molecules contained in each of a plurality of specimens;
an evaluating step that, by using an evaluation algorithm that evaluates properties of the specimens based on expression levels of nucleic acid molecules, evaluates properties of each of the plurality of specimens based on the expression levels of the nucleic acid molecules that were measured in the measuring step, and determines evaluation values; and
a detecting step that assigns ranks to the evaluation values of the plurality of specimens with respect to high/low levels, and, based on correlation between the ranks and the evaluation values of the plurality of specimens, detects an abnormality that has arisen in processes up to determination of the evaluation values.

2. The detection method of Claim 1, wherein, in the detecting step:
the evaluation values of the plurality of specimens are ranked in order from highest, and
a case in which an evaluation value of any specimen, whose rank is lower than a first threshold value, is a value that is greater than or equal to a second threshold value is detected as the abnormality.

3. The detection method of Claim 1, wherein, in the measuring step, the expression levels are measured for the plurality of specimens in a same process.

4. The detection method of Claim 1, further comprising a judging step that, in a case in which the abnormality is not detected in the detecting step, carries out, on the plurality of specimens, a judgment relating to the specimens based on the evaluation values determined by the evaluating step.

5. The detection method of Claim 2, wherein:
there are a plurality of patterns of combinations of the first threshold value and the second threshold value, and
in the detecting step, the abnormality is detected by using one pattern of the plurality of patterns.

6. The detection method of Claim 2, wherein, in the detecting step, the first threshold value is decided based on information relating to an origin source from which the plurality of specimens were taken, and the abnormality is detected.

7. A presentation device that, based on expression levels of nucleic acid molecules contained in each of a plurality of specimens, assigns ranks to evaluation values, by which properties of the specimens are evaluated, with respect to high/low levels, and presents a correlation between the ranks and the evaluation values of the plurality of specimens.

8. The presentation device of Claim 7, wherein the presentation device presents a distribution of the ranks and the evaluation values of the plurality of specimens, with respect to a first threshold value that is set for the ranks and a second threshold value that is set for the evaluation values.

9. The presentation device of Claim 7, wherein the presentation device presents accompanying information that accompanies judgment results of judgments relating to the specimens that were carried out based on the evaluation values.

10. A detection device comprising a processor, wherein the processor:
by using an evaluation algorithm that evaluates properties of specimens based on expression levels of nucleic acid molecules, evaluates properties of each of a plurality of specimens based on expression levels of the nucleic acid molecules that have been measured in advance, and determines evaluation values; and
assigns ranks to the evaluation values of the plurality of specimens with respect to high/low levels, and, based on correlation between the ranks and the evaluation values of the plurality of specimens, detects an abnormality that has arisen in processes up to determination of the evaluation values.

11. A detection system comprising:
a measurement unit that measures expression levels of a plurality of nucleic acid molecules contained in each of a plurality of specimens;
an evaluation unit that, by using an evaluation algorithm that evaluates properties of the specimens based on expression levels of nucleic acid molecules, evaluates properties of each of the plurality of specimens based on the expression levels of the nucleic acid molecules that were measured at the measurement unit, and determines evaluation values; and
a detector that assigns ranks to the evaluation values of the plurality of specimens with respect to high/low levels, and, based on correlation between the ranks and the evaluation values of the plurality of specimens, detects an abnormality that has arisen in processes up to determination of the evaluation values.

12. A detection program for causing a computer to execute detecting processing that:
by using an evaluation algorithm that evaluates properties of specimens based on expression levels of nucleic acid molecules, evaluates properties of each of a plurality of specimens based on expression levels of the nucleic acid molecules that have been measured in advance, and determines evaluation values; and
assigns ranks to the evaluation values of the plurality of specimens with respect to high/low levels, and, based on correlation between the ranks and the evaluation values of the plurality of specimens, detects an abnormality that has arisen in processes up to determination of the evaluation values.

13. A non-transitory recording medium on which is recorded a detection program for causing a computer to execute detecting processing that:
by using an evaluation algorithm that evaluates properties of specimens based on expression levels of nucleic acid molecules, evaluates properties of each of a plurality of specimens based on expression levels of the nucleic acid molecules that have been measured in advance, and determines evaluation values; and
assigns ranks to the evaluation values of the plurality of specimens with respect to high/low levels, and, based on correlation between the ranks and the evaluation values of the plurality of specimens, detects an abnormality that has arisen in processes up to determination of the evaluation values.
